# DEMANDE DE BREVET EUROPEEN

(11) **EP 1 192 930 A1**
(43) Date de publication de la demande: **03.04.2002**
(21) Numéro de dépôt: 01402444.2
(22) Date de dépôt: 24.09.2001
(51) Int. Cl.: A61K 7/043, A61K 7/00

(54) **Composition cosmétique filmogène**

(30) Priorité: 29.09.2000 FR 0012463
(71) Demandeur: L'OREAL, 75008 Paris (FR)
(72) Inventeur: Bernard, Pascale, 94370 Sucy-en-Brie (FR); Blin, Xavier, 75006 Paris (FR)
(74) Mandataire: Kromer, Christophe

(57) **Abrégé**

L'invention a pour objet une composition cosmétique comprenant :
- une dispersion aqueuse de particules de polymère, le polymère ayant une température de transition vitreuse (Tg) allant de 35°C à 80°C et une température minimale de filmification (TMF) telle que Tg - TMF ≥ 8°C,
- un premier solvant organique ayant un poids moléculaire inférieur ou égal à 200 et un point d'ébullition, mesuré à pression ambiante, allant de 100 °C à 300 °C, et
- un deuxième solvant organique ayant un poids moléculaire supérieur à 200 et un point d'ébullition, mesuré à pression ambiante, supérieur ou égal à 120 °C.

La composition est destinée au maquillage et au soin des matières kératiniques

## Description

La présente invention a pour objet une composition cosmétique filmogène comprenant une dispersion aqueuse de polymère et des solvants organiques utilisable notamment comme composition de maquillage ou de soin des matières kératiniques telles que la peau, y compris les lèvres, les ongles, les cils, les sourcils, les cheveux d'êtres humains. L'invention se rapporte aussi à l'utilisation de cette composition pour le maquillage ou le soin cosmétique des matières kératiniques. Plus spécialement, la composition est un vernis à ongles.

La composition de vernis à ongles peut être employée comme base pour vernis, comme produit de maquillage des ongles, comme composition de finition, encore appelée "top-coat" en terminologie anglosaxonne, à appliquer sur le produit de maquillage des ongles ou bien encore comme produit de soin cosmétique des ongles. Ces compositions peuvent s'appliquer sur les ongles d'êtres humains ou sur des faux ongles.

Les compositions cosmétiques filmogènes, notamment les vernis à ongles, contiennent généralement un polymère comme agent de formation du film et éventuellement des matières colorantes. Ces compositions doivent présenter des propriétés cosmétiques comme une filmification rapide pour obtenir un film non collant au toucher et pouvant résister à des agressions mécaniques telles que les chocs ou les frottements ou bien encore résister aux solvants tels que l'eau. Le film doit être brillant pour conférer un effet esthétique visuel agréable. Le film de vernis doit être bien adhérent sur les matières kératiniques et présenter une bonne tenue sans altération des propriétés initales au cours du temps, notamment après 2 jours. Enfin, le film doit pouvoir se démaquiller facilement avec les dissolvants classiques disponibles tels que des dissolvants à base d'acétone ou d'acétate d'éthyle.

Il est connu des compositions cosmétiques aqueuses comprenant un polymère en dispersion aqueuse. Très souvent, le polymère possède une température de transition vitreuse et une température de filmification voisines ; or un tel polymère ne permet pas d'obtenir un film séchant rapidement, non collant et présentant une bonne dureté. De plus, il est difficile de démaquiller correctement le film avec les dissolvants classiques.

Le but de la présente invention est de proposer une composition cosmétique filmogène à milieu aqueux, en particulier de vernis à ongles, formant un film présentant de bonnes propriétés telles que l'adhérence et la brillance, n'étant pas collant et pouvant se démaquiller avec les dissolvants classiques à base d'acétone et/ou d'acétate d'éthyle.

Le demandeur a constaté qu'une telle composition pouvait être obtenue en employant un polymère en dispersion aqueuse particulier associé à des solvants sélectionnés.

Plus précisément, l'invention a pour objet une composition cosmétique comprenant une dispersion aqueuse de particules de polymère, caractérisée par le fait que :
- le polymère a une température de transition vitreuse (Tg) allant de 35°C à 80°C et une température minimale de filmification (TMF) telle que Tg - TMF ≥ 8°C,
la composition comprenant, en outre :
- au moins un premier solvant organique ayant un poids moléculaire inférieur ou égal à 200 et un point d'ébullition, mesuré à pression ambiante, allant de 100 °C à 300 °C, et
- au moins un deuxième solvant organique ayant un poids moléculaire supérieur à 200 et un point d'ébullition, mesuré à pression ambiante, supérieur ou égal à 120 °C.
   L'invention a également pour objet un procédé cosmétique de maquillage et/ou de soin des matières kératiniques, en particulier des ongles, comprenant l'application sur les matières kératiniques d'une composition telle que définie précédemment.
   L'invention a encore pour objet l'utilisation d'une composition telle que définie précédemment pour l'obtention d'un film démaquillable à l'acétone et/ou à l'acétate d'éthyle et/ou adhérent sur l'ongle et/ou brillant.
   L'invention a aussi pour objet l'utilisation
- d'une dispersion aqueuse de polymère, le polymère ayant une température de transition vitreuse (Tg) allant de 35°C à 80°C et une température minimale de filmification (TMF) telle que Tg - TMF ≥ 8°C,
- d'un premier solvant organique ayant un poids moléculaire inférieur ou égal à 200 et un point d'ébullition, mesuré à pression ambiante, allant de 100 °C à 300 °C, et
- d'un deuxième solvant organique ayant un poids moléculaire supérieur à 200 et un point d'ébullition, mesuré à pression ambiante, supérieur ou égal à 120 °C,
dans une composition cosmétique pour l'obtention d'un film démaquillable à l'acétone et/ou à l'acétate d'éthyle et/ou adhérent sur l'ongle et/ou brillant.

Le polymère en dispersion aqueuse présent dans la composition selon l'invention a une température de transition vitreuse (Tg) allant de 35°C à 80°C et une température minimale de filmification (TMF) telle que Tg - TMF ≥ 8°C ; cette règle doit être valable pour au moins une Tg lorsque le polymère a plusieurs Tg.

La température de transition vitreuse est définie pour le polymère en l'absence d'agent auxiliaire de filmification et est déterminée pour un film séché à température ambiante (23 °C) et à humidité relative ambiante (50 %).

De préference, la température de transition vitreuse (Tg) du polymère va de 40 °C à 65 °C.

Avantageusement, Tg - TMF ≥ 12°C, mieux Tg - TMF ≥ 16°C, et préférentiellement Tg - TMF ≥ 18°C. En particulier, Tg - TMF peut être inférieur ou égal à 25 °C.

Le polymère en dispersion aqueuse selon l'invention est caractérisé par sa TMF en l'absence d'agent auxiliaire de filmification et la température de transition vitreuse (Tg) du film obtenues après séchage. La TMF est la température limite au-delà de laquelle une dispersion aqueuse de polymère forme, en séchant dans des conditions déterminées au préalable, un film exempt de fissures. La détermination de la TMF est régie selon les conditions décrites dans DIN 53787. Au début de la détermination, la dispersion aqueuse de polymère a une teneur en matière sèche de polymère de 30 à 55 % en poids. Comme TMF, on utilise pour les besoins de l'invention la moyenne de 10 mesures effectuées indépendamment. La détermination se fait en l'absence d'agents auxiliaires de filmification, c'est-à-dire que la TMF est une caractéristique intrinsèque du polymère en dispersion. Il est évident que la composition cosmétique formulée peut contenir, si on le désire, des agents auxiliaires de filmification.

La détermination de la température de transition vitreuse (Tg) se fait sur calorimètre DSC usuel dans le commerce, avec un taux de chauffage de 20 °C pour un film sec d'épaisseur de 100 µm séché à 23 °C et à un taux d'humidité relative de 50 %.

La taille des particules de polymère en dispersion aqueuse peut aller de 50 à 200 nm, de préférence 80 à 150 nm. De telles particules permettent d'obtenir un film d'une brillance particulièrement élevée et durable.

Le polymère en dispersion aqueuse est généralement obtenu par polymérisation de monomères à insaturation éthylénique dans un système bi-phase contenant une phase continue aqueuse. Habituellement, on utilise un système initiateur hydrosoluble pour initialiser la polymérisation. En général, la phase aqueuse contient des émulsifiants et/ou des agents colloïdes de protection.

Les monomères à insaturation éthylénique utilisables sont par exemple les (méth)acrylates d'alkyle en C₁-C₃₀, les esters vinyliques, les monomères aromatiques vinyliques et leurs mélanges.

Les agents colloïdes de protection utilisés peuvent être les polymères et copolymères hydrophiles tels que les alcools polyvinyliques, les acides polyacryliques, les polyacrylamides, les polyvinylpyrrolidones, les polyesters à groupe sulfonate, les polyamides à groupe sulfonate, les polyuréthanes à groupe sulfonate, les polyesteramides à groupe sulfonate ou carboxylique. On utilise de préférence des colloïdes de protection hydrophiles comprenant des groupes ioniques ou ionogènes.

Le polymère en dispersion aqueuse particulièrement préféré contient du styrène et préférablement au moins un monomère choisi parmi le méthacrylate de méthyle, le (méth)acrylate de n-butyle, le (méth)acrylate de tert-butyle et de préférence au moins un monomère choisi parmi les acides acrylique, méthacrylique, crotonique, itaconique.

De préférence, le polymère contient au moins 0,2 % en poids, de préférence de 0,2 à 50 % en poids, mieux de 2 % à 50 %, en particulier de 0,6 % à 25 %, voire de 3 % à 25 % en poids, par rapport au poids, total de monomères, de monomère de formule (I) : 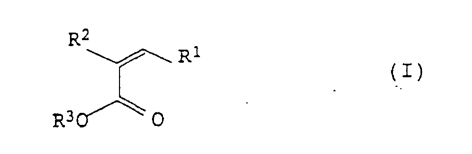 dans laquelle R¹ et R², indépendants l'un de l'autre, désignent un atome d'hydrogène ou un groupe méthyle, R³ désigne un radical alkyle ramifié, cyclique ou linéaire, en C₉-C₃₀, et particulièrement en C₁₂-C₂₂. R³ désigne de préférence un radical alkyle linéaire.

Comme monomères de formule (I), on peut citer les esters de l'acide (méth)acrylique et des alcools en C₁₂-C₂₂, comme le (méth)acrylate de lauryle, le (méth)acrylate de stéaryle, les esters de l'acide méthylméthacrylique et des alcools en C₁₂-C₂₂ ainsi que les esters de l'acide crotonique et des alcools en C₁₂-C₂₂.

De préférence, la proportion de styrène et de monomères de formule (I) dans ledit polymère, par rapport aux monomères totaux, va de 15 à 80 % en poids, et en particulier de 30 à 60 % en poids.

Le polymère en dispersion aqueuse (appelé polymère principal) est de préférence un polymère en émulsion comprenant des particules contenant plusieurs domaines de polymère, notamment des particules contenant plusieurs couches de polymère (on parle aussi de particules multicouches) ; ces particules peuvent contenir un ou plusieurs polymères, notamment au moins un premier polymère et au moins un second polymère, chaque polymère formant un domaine de polymère, notamment une couche de polymère.

Le premier polymère présent dans les particules du polymère principal peut être constitué de :
- 5 à 50 parties en poids, de préférence 8 à 30 parties en poids de monomères ayant au moins un groupe ionique ou ionogène,
- 50 à 95 parties en poids, de préférence 70 à 92 parties en poids de monomères neutres.

Le deuxième polymère est de préférence essentiellement constitué de monomères neutres.

Le polymère principal en émulsion peut être préparé par polymérisation en émulsion d'un mélange de monomères constituant le deuxième polymère, en présence du premier polymère. Ce dernier agit en règle générale comme colloïde de protection lors de la polymérisation en émulsion. On obtient ainsi des particules de polymère comprenant deux domaines de polymère, notamment deux couches de polymère (par exemple un coeur constitué du deuxième polymère et une écorce constituée du premier polymère).

Les termes « premier » et « deuxième » polymères sont utilisés dans la description dans un esprit de simplification.

Comme monomères comprenant des groupes ioniques ou ionogènes, on peut citer les monomères anioniques, cationiques ou amphotères.

Les groupes ionogènes des monomères peuvent être convertis en groupe ionique par protonation / déprotonation ou quaternisation.

Les monomères ioniques peuvent être neutralisés en totalité ou partiellement.

Selon un mode de réalisation de l'invention, le premier polymère peut comprendre des monomères anioniques et cationiques, ces deux types de monomères pouvant être présents en quantité équimolaires l'un par rapport à l'autre, ou bien l'un de ces deux types de monomères pouvant être en excès molaire par rapport à l'autre type de monomère de telle sorte que le premier polymère est anionique ou cationique. Ce premier polymère peut favoriser l'adhésion du polymère sur les matières kératiniques ou bien favoriser la stabilité de la dispersion aqueuse du polymère.

Comme monomères anioniques, ou acides, on peut utiliser des acides mono- ou dicarboxyliques à insaturation éthylénique, ayant de préférence 3 à 6 atomes de carbones, ainsi que des dérivés d'acide carboxylique polymérisables ou copolymérisables, comme les acides (méth)acryliques, crotoniques, maléiques et leurs anhydrides et monoesters, acide fumarique et monoester, acide itaconique ;
les monomères à insaturation éthylénique comprenant au moins un groupe acide sulfonique comme l'acide styrène sulfonique, l'acide vinylsulfonique, l'acide 2-acrylamido-2-méthyl-propanesulfonique ou leurs sels ;
les monomères à insaturation éthylénique comprenant au moins un groupe d'acide phosphonique ou phosphorique comme l'acide vinylphosphonique ou les monoesters d'acide phosphorique et d'alcools polymérisables comme par exemple le monoacrylate de butanediol ou le méthacrylate d'hydroxyéthyl.

Comme monomère anionique, on utilise de préférence les acides mono ou dicarboxyliques à insaturation éthylénique comme les acides acrylique, méthacrylique et crotonique.

Comme monomères cationiques, ou basiques, on peut utiliser les esters d'acide (méth)acrylique ou amides d'aminoalcools ou des diamines comme les (méth)acrylates de dialkylaminoalkyl ou les (méth)acrylamides de dialkylaminoalkyl comme le (méth)acrylate de N,N-diméthylaminoéthyl, le méthacrylate de N,N-diméthylaminoéthyl, l'acrylamide de N,N-diméthylaminopropyl, les dialkylaminostyrènes, comme par exemple le N,N-diméthylaminostyrène et le N,N diméthylaminométhylstyrène, la vinylpyridine comme la 4-vinylpyridine et la 2-vinylpyridine, le 1-vinylimidazol, ces monomères cationiques pouvant être quaternisés avec des réactifs connus de quaternisation tels que les alkylhalogénures, les benzylhalogenures, les dialkylsulfates.

Comme monomères amphotères, on peut utiliser le N-(3-sulfopropyl)-N-méthacryloyloxyéthyl-N,N-diméthylammonium-bétaine et le N-carboxyméthyl-N-méthacryloyloxyéthyl-N,N-diméthylammonium bétaine.

Dans les groupes ioniques, les groupes acides ou les groupes amine tertiaire peuvent être modifiés par salification ou réaction de quaternisation.

Les monomères neutres sont des monomères sans groupes ioniques ou ionogènes. Les monomères neutres peuvent être également les monomères de formule (I) indiquée précédemment. Le polymère en émulsion contient en général d'autres monomères neutres, différents de ceux de formule (I), qui peuvent être soit des monomères principaux ou soit des comonomères.

Comme monomères neutres principaux, on peut citer par exemple :
les (méth)acrylates d'alkyle en C₁-C₈ comme le (méth)acrylate de méthyle, le (méth)acrylate d'éthyle, le (méth)acrylate de propyle, le (méth)acrylate de n-butyl, l'acrylate de 2-éthyl hexyle ;
les esters vinyliques de l'acide carboxylique en C₁-C₁₈, en particulier en C₁-C₈, comme l'acétate de vinyle, le propionate de vinyle, le laurate de vinyle, le néodécanoate de vinyle;
les monomères vinyliques aromatiques, comme le styrène, l'α-méthylstyrène, l'α-butylstyrène, les alkyl (C₁-C₁₀) styrènes comme le 4-butylstyrène, le 4-décylstyrène , l'hydroxystyrène comme le 4-hydroxy styrène,
ou leurs mélanges.

Comme monomères neutres principaux, on peut citer également les oléfines aliphatiques ayant de 2 à 8 atomes carbone et une ou deux doubles liaisons éthyléniques comme le butadiène, l'isoprène et le chloroprène, ainsi que l'éthylène, le propylène et l'isobutylène. On utilise de préférence des oléfines aliphatiques contenant une seule double liaison éthylénique.

Les monomères neutres principaux préférés sont choisis parmi le styrène, le méthacrylate de méthyle, le (méth)acrylate de n-butyle, le (méth)acrylate de tert-butyle, le (méth)acrylate d'iso-butyle.

Les comonomères neutres peuvent être choisis parmi les monomères contenant des groupes hydroxyles comme les (méth)acrylates d'hydroxyalkyle en C₁-C₆ tels que le (méth)acrylate d'hydroxypropyl ou le (méth)acrylate d'hydroxyéthyl, les amides ou amides substitués d'acides mono ou dicarboxyliques à insaturation éthylénique, tels que l'acrylamide, le méthacrylamide, le N-méthylolacrylamide, le N-méthylolméthacrylamide, les (méth)acrylamides de N-alkyl en C₁-C₁₀, les (méth)acrylamides de N,N-dialkyle en C₁-C₁₀. Les monomères réticulants, notamment ceux comportant deux groupes vinyle, peuvent être utilisés conjointement, ceux-ci étant présents de préférence dans le deuxième polymère.

Comme comonomère neutre, on peut également citer les nitriles et les halogénures de vinyle. Comme exemple de monomères nitriles, on peut citer l'acrylonitrile et le méthacrylonitrile. Les halogénures de vinyle sont des composés à insaturation éthylénique substitués par un atome d'halogène tel que le chlore, le fluor et le brome. L'halogénure de vinyle peut être choisi parmi le chlorure de vinyle, le chlorure de vinylidène, le fluorure de vinylidène.

Le deuxième polymère est surtout constitué de monomères neutres. Il est constitué de préférence de 60 à 100 % en poids de monomères neutres principaux cités précédemment et de 0 à 40 % de parties en poids de monomères différents des dits monomères neutres principaux, tels que les comonomères neutres cités précédemment. Le deuxième polymère peut comprendre également au moins un monomère ionique, tels que ceux cités précédemment, en une proportion pondérérale de préférence inférieure à 5 % en poids.

Avantageusement, le polymère en dispersion aqueuse comprend un premier polymère constitué de :
- 5 à 40 parties en poids de monomères comprenant au moins un groupe ionique ou ionogène,
- 2 à 50 parties en poids, de préférence 10 à 30 parties en poids, de monomères de formule (I) indiquée précédemment,
- 10 à 93 parties en poids, de préférence 40 à 85 parties en poids, de monomères neutres choisis parmi les (méth)acrylates d'alkyle en C₁-C₈, les esters vinyliques d'acides carboxyliques en C₁-C₁₈, les monomères aromatiques vinyliques ou leurs mélanges, et
- 0 à 40 parties en poids de monomères différents de ceux mentionnés précédemment,
et un deuxième polymère constitué de :
- 60 à 100 parties en poids de monomères neutres choisis parmi les (méth)acrylates d'alkyl en C₁-C₈, les esters vinyliques d'acides carboxyliques en C₁-C₁₈, les monomères aromatiques vinyliques ou leurs mélanges et
- 0 à 40 parties en poids de monomères différents.

Dans les particules du polymère en dispersion aqueuse, le rapport pondéral entre le premier polymère et le deuxième polymère va de préférence de 10:90 à 60:40, en particulier 30:70 à 50:50.

Le poids moléculaire moyen (Mw) du premier polymère est de préférence supérieur à 10 000, ou va notamment de 20 000 à 200 000 (déterminé par chromatographie avec polystyrène comme standard et tétrahydrofurane comme éluant).

Le premier polymère peut être obtenu par n'importe quel procédé de polymérisation, mais de préférence par polymérisation en solution.

Comme solvant pour la polymérisation en solution du premier polymère, on peut utiliser les solvants ayant un point d'ébullition inférieur à 100°C à 100000 Pa (1 bar) ou ceux qui forment un azéotrope avec l'eau et peuvent être facilement isolés, par distillation, de la dispersion aqueuse de polymère ou de la solution de polymère. On peut également ajouter au solvant, dans des cas particuliers, des agents auxiliaires de filmification tels que ceux mentionnés précédemment.

On peut citer, comme solvants, les alcools ou cétones ayant jusqu'à 8 atomes de carbone comme le butanol, l'isobutanol, le propanol, l'éthanol, le méthanol et la méthyléthylcétone.

La polymérisation des monomères à insaturation éthylénique peut s'effectuer par polymérisation anionique ou de préférence radicalaire, notamment en présence d'initiateurs radicalaires tels que les peroxydes. La teneur en initiateur radicalaire peut aller de 0,2 à 5 % en poids, et mieux de 0,5 à 3 % en poids par rapport aux monomères. La température de polymérisation peut être choisie dans une gamme de température allant de 50 à 150°C, et de préférence de 70 à 130°C. Le cas échéant, on peut ajouter des régulateurs, comme par exemple le mercaptoéthanol, le dodécylmercaptan tertiaire, l'éthylhexylthioglycolate ou le sulfure de diisopropylxanthogène, en une teneur pouvant aller de 0 % et 3 % en poids par rapport aux poids de monomères.

La préparation du premier polymère peut s'effectuer en une ou plusieurs étapes. En particulier, on peut par exemple tout d'abord fabriquer un polymère ayant une teneur élevée en acide, suivi d'un polymère ayant une plus faible teneur en acide, comme il est par exemple décrit dans EP-A-320865. Les monomères peuvent être introduits lors de la polymérisation ou de préférence, rajoutés en continu.

Le premier polymère est obtenu en dispersion ou de préférence en solution dans le solvant organique. La teneur en matière sèche de premier polymère va de préférence de 50 à 95 % en poids, et notament de 60 à 85 % en poids.

Pour la fabrication du polymère en émulsion, la polymérisation en émulsion est effectuée en présence du premier polymère. La polymérisation en émulsion s'effectue en général à une température allant de 30 °C à 95°C en présence d'un initiateur hydrosoluble. On peut citer comme initiateurs le persulfate de sodium, le persulfate de potassium et le persulfate d'ammonium, le tert-butylhydropéroxyde, les combinaisons azoïques hydrosolubles ou les systèmes initiateurs redox. Si l'on utilise l'H₂O₂ en tant qu'initiateur, on utilise conjointement de faibles quantités de sels de métaux lourds, comme les sels de Cu(ll)- ou Fe(III)-.

Le premier polymère peut être introduit dans l'eau ou dans un autre milieu aqueux et/ou être incorporé à l'eau avec les monomères du deuxième polymère en cours de polymérisation pendant la polymérisation en émulsion. Préférablement, l'eau est ajoutée à la solution de polymère et ensuite, le solvant organique utilisé est éliminé par distillation.

Dans la mesure où le premier polymère comporte des groupes acides ou anhydrides, ceux ci sont neutralisés avant ou pendant l'introduction dans la phase aqueuse, partiellement ou totalement, à l'aide d'agents de neutralisation.

Les agents de neutralisation adaptés sont d'une part des bases minérales comme le carbonate de sodium ou le carbonate de potassium ainsi que l'ammoniaque, d'autre part les bases organiques comme les aminoalcools tels que le 2-amino-2-méthyl-1-propanol (AMP), la triéthanolamine, la triisopropanolamine (TIPA), la monoéthanolamine, la diéthanolamine, la tri[(2-hydroxy)-1-propyl]amine, le 2-amino-2-méthyl-1,3-propanediol (AMPD) ou le 2-amino-2-hydroxyméthyl-1,3-propanediol ainsi que les diamines, comme la lysine.

Pour la polymérisation en émulsion, il n'est en général pas nécessaire d'utiliser d'autres émulsifiants, colloïdes de protection ou auxiliaires de dispersion en plus du premier polymère ; ceux-ci peuvent toutefois être rajoutés.

On peut utiliser, pendant et après la polymérisation en émulsion, des additifs pour abaisser la viscosité. Ceux-ci sont de préférence des sels d'acides organiques ou de bases tels que l'hydrochlorure de lysine et le citrate de sodium.

Dans la composition selon l'invention, le polymère en dispersion aqueuse peut être présent en une teneur allant de 0,1 à 60 % en poids, par rapport au poids total de la composition, de préférence de 1 % à 50 % en poids et mieux de 5 % à 40 % en poids.

Le premier solvant organique présent dans la composition selon l'invention, appelé également agent de coalescence, favorise la coalescence des particules de polymère en dispersion aqueuse. Le premier solvant organique a un poids moléculaire inférieur ou égal à 200, de préférence inférieur ou égal à 160 ; en particulier, le poids moléculaire va de 50 à 200, et mieux de 50 à 160. Son point d'ébullition peut aller 120 °C à 250 °C, et mieux de 130 °C à 230 °C.

Comme exemple de premier solvant organique, on peut citer :
les éthers de propylène glycol tels que le n-butyl éther de propylène glycol, le t-butyl éther de propylène glycol, le n-propyl éther de propylène glycol, le phényl éther de propylène glycol,
les éthers de dipropylène glycol tels que le n-butyl éther de dipropylène glycol, le méthyl éther de dipropylène glycol, le t-butyl éther de dipropylène glycol, le n-propyl éther de dipropylène glycol,
l'acétate du méthyl éther de propylène glycol, le diacétate de propylène glycol,
le lactate de méthyle, le lactate d'éthyle, le lactate d'isopropyle, le lactate de butyle.

Le premier solvant organique peut être présent dans la composition selon l'invention en une quantité suffisante pour obtenir un film déposé sur les matières kératiniques, notamment sur les ongles ; en particulier, la teneur en premier solvant organique dans la composition peut aller de 0,05 % à 10 % en poids, par rapport au poids total de la composition, et mieux de 0,1 % à 8 % en poids.

Le deuxième solvant organique présent dans la composition, appelé également plastifiant, permet de plastifier le polymère en dispersion aqueuse. De préférence, le deuxième solvant organique a un poids moléculaire supérieur à 200, de préférence supérieur ou égal à 230, et mieux supérieur ou égal à 250. En particulier, le poids moléculaire peut être inférieur ou égal à 600, de préférence inférieur ou égal à 500.

Son point d'ébullition est de préférence supérieur ou égal à 140 °C, et mieux supérieur ou égal à 160 °C ; il est notamment inférieur à 500 °C.

Comme exemple de deuxième solvant organique, on peut citer :
les adipates tels que l'adipate de diéthyle, l'adipate de dibutyle, l'adipate de diisobutyle, l'adipate de di-isopropyle,
les sébacates tels que le sébacate de diméthyle, le sébacate de diéthyle, le sébacate de dibutyle,
les citrates tels que le citrate de triéthyle, le citrate d'acétyl triéthyle, le citrate d'acétyl tributyle,
les phtalates tels que le phtalate de diéthyle, le phtalate de dibutyle, le phtalate de dioctyle.

Le deuxième solvant organique peut être présent dans la compostion selon l'invention en une quantité suffisante pour plastifier le film de polymère déposé sur les matières kératiniques. En particulier, la teneur en deuxième solvant organique dans la composition peut aller de 0,05 % à 20 % en poids, par rapport au poids total de la composition, et mieux de 0,1 % à 10 % en poids.

Avantageusement, la composition selon l'invention ne contient pas 7,50 % en poids d'éthoxydiglycol et moins de 0,15 % en poids de propylène glycol.

La composition selon l'invention peut en outre comprendre des matières colorantes telles que les colorants hydrosolubles, les matières colorantes pulvérulentes comme les pigments, les nacres, les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

La composition selon l'invention peut également comprendre un agent épaissant tels que les argiles gonflant dans l'eau comme les hectorites, les bentonites, les épaisssants associatifs tels que les polyuréthanes associatifs, les polymères acryliques associatifs, les épaississants cellulosiques hydrosolubles tels que l'hydroxyéthylcellulose. L'agent épaississant peut être présent en une teneur allant de 0,1 % à 5 % en poids, par rapport au poids total de la composition.

La composition selon l'invention peut en outre comprendre tout additif cosmétique connu de l'homme du métier comme étant susceptible d'être incorporé dans une telle composition, tels que les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

L'invention est illustrée plus en détail dans l'exemple suivant.

### Exemple 1 :

### a) Préparation du polymérisat constituant le premier domaine de polymère par polymérisation en solution.

Dans un ballon en verre, équipé d'un réfrigérant à reflux, d'un dispositif d'agitation à armatures, d'un entonnoir à brome et d'un bain d'huile thermostaté, on chauffe sous agitation à 85°C dans une atmosphère sous azote 136 g d'isopropanol. Puis, on ajoute en 5 heures un mélange comprenant 240 g de n-propanol et 21,3 g de ter-perpivalate de tert-butyle (à 75 %). 15 minutes après le début de l'étape précédente, on introduit pendant 3 heures 30 minutes un mélange de monomères comprenant, 14,4 g d'acide acrylique, 21,6 g d'acrylaye de n-butyle, 28,8 g d'acrylate de lauryle et 79,2 g de méthacrylate de méthyle. La solution de polymère est ensuite refroidie à 80 °C et est neutralisée en 30 minutes avec 75,6 g d'une solution aqueuse à 25 % en poids d'ammoniaque. On agite à nouveau pendant 30 minutes. La solution de polymère est dispersée par addition de 1200 g d'eau pendant une heure puis on chauffe le mélange à une température allant jusqu'à 100°C pour recueillir 800 g de distillat. On obtient ainsi une solution aqueuse de polymère contenant 24,9 % en poids de matières sèches. Ce polymère a une valeur K (3 g de matière sèche dans 100 ml d'acétone) égale à 34,2. La valeur K (ou constante de Fikentscher) est calculée à partir de la viscosité en solution du polymère et est expliquée dans la littérature spécialisée, par ex. H.G. Elias, macromolécule, vol 1, Hûthig & Wepf, Heidelberg 1990, page 98f.

### b) Préparation du polymérisat en plusieurs étapes par polymérisation en émulsion d'un mélange de monomères en présence du polymérisat constituant le premier domaine de polymère.

1285 g de solution de polymère obtenue à l'étape a) précédente est réchauffée à 85°C sous agitation, sous atmosphère azote. On incorpore ensuite en 10 minutes un mélange comprenant 38 g d'eau, 0,016 g de CUSO₄. 5 H₂O et 1,6 g d'hydrochlorure de L-lysine. A 85°C, on incorpore 26,7 g d'une solution aqueuse d'eau oxygénée à 12 % en poids dans l'eau. Puis on ajoute en 2 heures un mélange de monomères comprenant 280 g de styrène, 60 g d'acrylate de n-butyle et 140 g d'acrylate de tert-butyle ainsi que 6,4 g d'hydrochlorure de L-lysine et 53,6 g d'eau, et en 2 heures et demi 106, 7 g d'une solution aqueuse d'eau oxygénée à 12 % en poids dans l'eau. On agite ensuite 1 heure à 85°C et on laisse refroidir à température ambiante en ajoutant un mélange comprenant 120 g d'eau et 80 g de tensioactif siliconé (n° CAS 71965-38-3). Après refroidissement, on obtient une dispersion aqueuse de particules de polymère contenant 40,45 % de matière sèche. Le polymère a une température minimale de filmification de 27 °C et une température de transition vitreuse de 55 °C. Les particules du polymère ont une taille maximale de 85 nm et une taille moyenne arithmétique de 94 nm.

Pour mesurer la Tg, on réchauffe l'échantillon de polymère depuis la température ambiante jusqu'à 120°C, puis on refroidit à -60°C et on réchauffe à nouveau à 120°C. C'est au cours de l'étape de réchauffement de -60°C à 120°C qu'on mesure la Tg. Toutes les étapes de réchauffement et de refroidissement se déroulent à une vitesse de chauffe de 20°C/mn.

La répartition de la taille des particules est déterminée avec un spectroscope, modèle Autosizer 2c de la société Malvern. On obtient, grâce à cet appareil, une détermination du volume total de toutes les particules d'une classe par rapport au diamètre des particules.

### c) On a préparé un vernis à ongles ayant la composition suivante :

- dispersion aqueuse de polymère de l'exemple 1b 35 g
- sébacate de diéthyle 1.5 g
- acétate du méthyl éther de propylène glycol 0.5 g
- n-butyl éther de propylène glycol 3 g
- silicate de magnésium et d'aluminium (laponite XLS de la société Laporte) 0.9 g
- pigments 1.5 g
- eau qsp 100 g

On obtient un vernis à ongles qui s'applique facilement sur les ongles et forme après séchage un film brillant, non collant. Le vernis se démaquille aisément avec le démaquillant vendu sous la dénomination Lancôme Douceur.

## Revendications

1. Composition cosmétique comprenant une dispersion aqueuse de particules de polymère, **caractérisée par le fait que** :
- le polymère a une température de transition vitreuse (Tg) allant de 35°C à 80°C et une température minimale de filmification (TMF) telle que Tg - TMF ≥ 8°C,
et que la composition comprend, en outre :
- au moins un premier solvant organique ayant un poids moléculaire inférieur ou égal à 200 et un point d'ébullition, mesuré à pression ambiante, allant de 100 °C à 300 °C, et
- au moins un deuxième solvant organique ayant un poids moléculaire supérieur à 200 et un point d'ébullition, mesuré à pression ambiante, supérieur ou égal à 120 °C.

2. Composition selon la revendication 1, **caractérisée par le fait que** le polymère est tel que Tg - -TMF ≥ 12°C, et mieux Tg - TMF ≥ 16°C

3. Composition selon la revendication 1 ou 2, **caractérisée par le fait que** les particules de polymère ont une taille allant de 50 à 200 nm.

4. Composition selon l'une quelconque des revendications précédentes, **caractérisée par le fait que** le polymère en dispersion aqueuse est obtenu par polymérisation de monomère à indsaturation éthylénique choisi dans le groupe formé par les (méth)acrylates d'alkyle en C₁-C₃₀, les esters vinyliques, les monomères aromatiques vinyliques et leurs mélanges.

5. Composition selon l'une quelconque des revendications précédentes, **caractérisée par le fait que** le polymère en dispersion aqueuse contient du styrène.

6. Composition selon la revendication 5, **caractérisée par le fait que** le polymère contient également un monomère choisi dans le groupe formé par le méthacrylate de méthyle, le (méth)acrylate de n-butyle, le (méth)acrylate de tert-butyle.

7. Composition selon la revendication 5 ou 6, **caractérisée par le fait que** le polymère en dispersion aqueuse contient un monomère choisi dans le groupe formé par l'acide acrylique, l'acide méthacrylique, l'acide crotonique, l'acide itaconique.

8. Composition selon l'une quelconque des revendications précédentes, **caractérisée par le fait que** le polymère en émulsion contient au moins 0,2 % en poids, par rapport au poids total de monomères, d'un monomère de formule (I) : 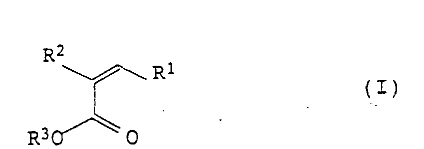 dans laquelle R¹ et R², indépendants l'un de l'autre, désignent un atome d'hydrogène ou un groupe méthyle, et R³ désigne un radical alkyle en C9-C30.

9. Composition selon la revendication 8, **caractérisée par le fait que** R³ désigne un radical en C₁₂-C₂₂.

10. Composition selon l'une des revendications 8 ou 9, **caractérisée par le fait que** le monomère de la formule (I) est choisi dans le groupe formé par le (méth)acrylate de lauryle et le (méth)acrylate de stéaryle.

11. Composition selon la revendication 5 et l'une des revendications 8 à 10, **caractérisée par le fait que** la proportion de styrène et de monomères de formule (I) dans le polymère en dispersion aqueuse, par rapport aux monomères totaux, va de 15 à 80 % en poids, et en mieux de 30 à 60 % en poids.

12. Composition selon l'une quelconque des revendications précédentes, **caractérisée par le fait que** les particules de polymère sont des particules contenant plusieurs domaines de polymère, qui comprend au moins un premier polymère et au moins un deuxième polymère,
le premier polymère étant constitué de :
- 5 à 50 parties en poids de monomères ayant au moins un groupe ionique ou ionogène, et
- 50 à 95 parties en poids de monomère neutres,
et le deuxième polymère étant essentiellement constitué de monomères neutres.

13. Composition selon la revendication 12, **caractérisée par le fait que** les monomères possédant un groupe ionique ou ionogène sont choisis dans le groupe formé par les acides mono- ou dicarboxyliques à insaturation éthylénique, les monomères à insaturation éthylénique comprenant au moins un groupe acide sulfonique, les monomères à insaturation éthylénique comprenant au moins un groupe d'acide phosphonique ou phosphorique.

14. Composition selon la revendication 12 ou 13, **caractérisée par le fait que** les monomères ioniques sont totalement ou partiellement neutralisés.

15. Composition selon l'une quelconque des revendications 12 à 14, **caractérisée par le fait que** le premier polymère est constitué de :
- 5 à 40 parties en poids de monomères comprenant au moins un groupe ionique ou ionogène,
- 2 à 50 parties en poids de monomères de formule (I),
- 10 à 93 parties en poids de monomères neutres choisis parmi les (méth)acrylates d'alkyle en C₁-C₈, les esters vinyliques d'acides carboxyliques en C₁-C₁₈, les monomères aromatiques vinyliques ou leurs mélanges, et
- 0 à 40 parties en poids de monomères différents de ceux mentionnés précédemment,
et le deuxième polymère est constitué de :
- 60 à 100 parties en poids de monomères neutres choisis parmi les (méth)acrylates d'alkyl en C₁-C₈, les esters vinyliques d'acides carboxyliques en C₁-C₁₈, les monomères aromatiques vinyliques ou leurs mélanges, et
- 0 à 40 parties en poids de monomères différents.

16. Composition selon l'une quelconque des revendications 12 à 15, **caractérisée par le fait que** le rapport pondéral entre le premier polymère et le second polymère va de 10:90 à 60:40.

17. Composition selon l'une quelconque des revendications précédentes, **caractérisée par le fait que** le polymère est présent en une teneur allant de 0,1 % à 60 % en poids, par rapport au poids total de la composition, et mieux de 1 % à 50 % en poids.

18. Composition selon l'une quelconque des revendications précédentes, **caractérisée par le fait que** le premier solvant organique a un point d'ébullition allant de 120 °C à 250 °C, et mieux de 130 °C à 230 °C.

19. Composition selon l'une quelconque des revendications précédentes, **caractérisée par le fait que** le premier solvant organique est choisi dans le groupe formé par le n-butyl éther de propylène glycol, le t-butyl éther de propylène glycol, le n-propyl éther de propylène glycol, le phényl éther de propylène glycol, le n-butyl éther de dipropylène glycol, le méthyl éther de dipropylène glycol, le t-butyl éther de dipropylène glycol, le n-propyl éther de dipropylène glycol, l'acétate du méthyl éther de propylène glycol, le diacétate de propylène glycol, le lactate de méthyle, le lactate d'éthyle, le lactate d'isopropyle, le lactate de butyle.

20. Composition selon l'une quelconque des revendications précédentes, **caractérisée par le fait que** le premier solvant organique est présent en une teneur allant de 0,05 % à 10 % en poids, par rapport au poids total de la composition, et de préférence allant de 0,1 % à 8 % en poids.

21. Composition selon l'une quelconque des revendications précédentes, **caractérisée par le fait que** le deuxième solvant organique a un point d'ébullition supérieur ou égale à 140 °C, et mieux supérieur ou égal à 160 °C.

22. Composition selon l'une quelconque des revendications précédentes, **caractérisée par le fait que** le deuxième solvant organique est choisi dans le groupe formé l'adipate de diéthyle, l'adipate de dibutyle, l'adipate de di-isobutyle, l'adipate de diisopropyle, le sébacate de diméthyle, le sébacate de diéthyle, le sébacate de dibutyle, le citrate de triéthyle, le citrate d'acétyl triéthyle, le citrate d'acétyl tributyle, le phtalate de diéthyle, le phtalate de dibutyle, le phtalate de dioctyle.

23. Composition selon l'une quelconque des revendications précédentes, **caractérisée par le fait que** le deuxième solvant organique est présent en une teneur allant de 0,05 % à 20 % en poids, par rapport au poids total de la composition, et mieux de 0,1 % à 10 % en poids.

24. Composition selon l'une quelconque des revendications précédentes, **caractérisée par le fait qu'**elle comprend un additif cosmétique choisi parmi les matières colorantes, les agents épaississants, les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

25. Composition selon la revendication 24, **caractérisée par le fait que** les matières colorantes sont choisies dans le groupe formé par les colorants hydrosolubles, les pigments, les nacres, les paillettes.

26. Composition selon la revendication 24 ou 25, **caractérisée par le fait que** les matières colorantes sont présentes en une teneur allant de 0,01 % à 50 % en poids, par rapport au poids de la composition, de préférence de 0,01 % à 30 % en poids.

27. Composition selon la revendication 24, **caractérisée par le fait que** l'agent épaissant est choisi dans le groupe formé par les argiles gonflant dans l'eau, les épaisssants associatifs, les épaississants cellulosiques hydrosolubles.

28. Composition selon la revendication 24 ou 25, **caractérisée par le fait que** l'agent épaississant est présent en une teneur allant de 0,1 % à 5 % en poids, par rapport au poids total de la composition.

29. Procédé cosmétique de maquillage et/ou de soin des matières kératiniques, en particulier des ongles, comprenant l'application sur les matières kératiniques d'une composition selon l'une quelconque des revendications précédentes.

30. Utilisation d'une composition selon l'une quelconque des revendications 1 à 28 pour l'obtention d'un film démaquillable à l'acétone et/ou à l'acétate d'éthyle et/ou l'obtention d'un film adhérent sur l'ongle et/ou brillant.

31. Utilisation
- d'une dispersion aqueuse de polymère, le polymère ayant une température de transition vitreuse (Tg) allant de 35°C à 80°C et une température minimale de filmification (TMF) telle que Tg - TMF ≥ 8°C,
- d'un premier solvant organique ayant un poids moléculaire inférieur ou égal à 200 et un point d'ébullition, mesuré à pression ambiante, allant de 100 °C à 300 °C, et
- d'un deuxième solvant organique ayant un poids moléculaire supérieur à 200 et un point d'ébullition, mesuré à pression ambiante, supérieur ou égal à 120 °C,
dans une composition cosmétique, pour l'obtention d'un film démaquillable à l'acétone et/ou à l'acétate d'éthyle et/ou d'un film adhérent sur l'ongle et/ou brillant.
